Europäisches Patentamt

European Patent Office

Office européen des brevets

(11) Publication number: 0 219 426
A2

# EUROPEAN PATENT APPLICATION

(21) Application number: 86402209.0

(22) Date of filing: 06.10.86

(51) Int. Cl.⁴: C 08 B 13/00
A 61 K 9/36, A 61 K 9/62
C 08 B 11/20

(30) Priority: 07.10.85 JP 223252/85

(43) Date of publication of application:
22.04.87 Bulletin 87/17

(84) Designated Contracting States:
BE CH DE FR GB IT LI SE

(71) Applicant: Shin-Etsu Chemical Co., Ltd.
6-1, Ohtemachi 2-chome
Chiyoda-ku Tokyo 100(JP)

(72) Inventor: Chiba, Tohru
14-45, Gochi, 2-chome
Joetsu-shi Niigata-ken(JP)

(72) Inventor: Sekigawa, Fujio
7E-10, Sanai-cho
Joetsu-shi Niigata-ken(JP)

(72) Inventor: Muto, Hiroaki
2-14-45, Gochi
Joetsu-shi Niigata-ken(JP)

(72) Inventor: Maruyama, Kazumasa
1-4-15, Minato-machi, 2-chome
Joetsu-shi Niigata-ken(JP)

(74) Representative: Armengaud Ainé, Alain et al,
Cabinet ARMENGAUD AINE 3 Avenue Bugeaud
F-75116 Paris(FR)

(54) A method for the preparation of an acidic dicarboxylic acid ester of cellulose ether.

(57) A hydroxypropoxyl-containing cellulose ether, e.g. hydroxypropyl cellulose and hydroxypropyl methyl cellulose, is esterified into an acidic dicarboxylic acid ester useful as a enteric coating material of solid medicament forms. The esterification reaction of 100 parts by weight of the cellulose ether with phthalic or succinic anhydride optionally combined with acetic anhydride is performed in 200 to 700 parts by weight of acetic acid as a solvent in the presence of 50 to 150 parts by weight of an alkali metal acetate, e.g. sodium acetate. The cellulose ether product is so tough and resistant against attack of a simulated gastric juice that the solid medicament forms coated therewith are imparted with highly reliable enterosolubility.

—1—

# A METHOD FOR THE PREPARATION OF AN ACIDIC DICARBOXYLIC ACID ESTER OF CELLULOSE ETHER

The present invention relates to a method for the preparation of an acidic dicarboxylic acid ester of a cellulose ether or, more particularly, to a method for the preparation of such an acidic ester of a cellulose ether by esterifying a hydroxypropoxyl-containing cellulose ether with a dicarboxylic acid anhydride in an object to provide an enterosoluble film-coating material on solid medicament forms having resistance against a simulated gastric juice.

As is well known, acidic dicarboxylic acid esters of a cellulose ether having hydroxypropoxyl groups are soluble generally in several organic solvents and also soluble in an alkaline aqueous solution due to the acidic nature thereof by the carboxyl groups. Therefore, such a material is widely used as an enterosoluble film-coating material on various solid medicament forms such as tablets, granules and pills. Typical products of acidic dicarboxylic acid esters of cellulose ethers include acidic phthalic acid esters of hydroxyalkyl alkyl celluloses and hydroxyalkyl celluloses disclosed in Japanese Patent Publications 47-6435 and 51-48515, acidic succinic acid esters of similar cellulose ethers disclosed in Japanese Patent Publication 48-19552 and mixed esters of similar cellulose ethers with succinic acid and aliphatic monocarboxylic acid disclosed in Japanese Patent Publication 57-25008.

When a solid medicament form coated with such an alkali-soluble cellulose ether derivative is orally administrated, the coating film is not affected by the acidic gastric juice in the stomach but readily dissolved in the alkaline intestinal juice when it has reached the intestine to release the medically active ingredients contained therein. When an improperly prepared acidic dicarboxylic acid ester of a cellulose ether having hydroxypropoxyl groups is used as an enterosoluble film-coating material, drawbacks are sometimes caused that cracks are formed in the coating film already in a simulated gastric juice, which is specified as the first fluid in the Japanese Pharmacopoeia, in the course of the disintegration test so that the coated solid medicament form can no longer be enterosoluble.

Accordingly, an object of the present invention is to provide an acidic dicarboxylic acid ester of a cellulose ether having hydroxyalkyl groups, which is capable of giving a very reliable enterosoluble film-coating on various solid medicament forms without being affected by the first fluid of the pharmacopoeia used in the disintegration test.

Thus, the method of the present invention for the preparation of an acidic dicarboxylic acid ester of a cellulose ether comprises reacting (a) a cellulose ether having hydroxypropoxyl groups as the ether-forming groups, of which a 2 % by weight aqueous solution has a viscosity of at least 5 centipoise at 20 °C, with (b) a dicarboxylic acid anhydride or a mixture thereof with an anhydride of an aliphatic monocarboxylic acid in the presence of (c) a combination of an alkali metal acetate and acetic acid.

As is described in the above given summary, the base material or starting material in the inventive method is a cellulose ether having hydroxypropoxyl groups as the ether-forming groups. The cellulose ether should be soluble in water or in several organic solvents. Exemplary of the cellulose ether are hydroxypropyl cellulose, hydroxypropyl methyl cellulose, hydroxypropyl ethyl cellulose and the like. The hydroxyalkoxyl groups in the cellulose ether must be hydroxypropoxyl since a cellulose ether having hydroxyalkoxyl groups other than hydroxypropoxyl cannot give an acidic dicarboxylic acid ester having good solubility in organic solvents so that difficulties are encountered in the coating works of solid medicament forms using the same. In this regard, the cellulose ether used as the base material of the inventive method should desirably have at least 4 % by weight of the hydroxypropoxyl groups. The cellulose ether should have such a degree of polymerization that a 2 % by weight aqueous solution thereof has a viscosity of at least 5 centipoise at 20 °C (the viscosity value of a cellulose ether hereinbelow mentioned always refers to the value at 20 °C measured of a 2 % by weight aqueous solution). This limitation is important in order that the solid medicament form coated with the acidic dicarboxylic acid ester of the cellulose ether is stable in the first fluid of the pharmacopoeia in the disintegration test without causing crack formation in the coating film or disintegration of the solid medicament form. Although the viscosity of the aqueous solution of the cellulose ether is not particularly limitative in respect of the upper limit, it is advantageous that the hydroxypropoxyl-containing cellulose ether should have a viscosity not exceeding 70 centipoise because the esterification reaction of a cellulose ether having a higher viscosity requires a large volume of acetic acid as the reaction medium for the esterification reaction with a decrease in the % utilization of the esterifying reagent in addition to the problem that the coating works using such a product must be conducted by use of a large volume of an organic solvent due to the increased viscosity of the solution of the product.

Following is a presumable mechanism by which the esterified cellulose ether product obtained by the inventive method can provide an enterosoluble coating film on solid medicament forms capable of withstanding the attack of the first fluid of the pharmacopoeia in the disintegration test. As is generally understood, a film formed of a polymeric material may have increased toughness as the average molecular weight thereof is increased or have increased fragility as the average molecular weight thereof is decreased. This trend is held also in cellulose ethers. When an acidic dicarboxylic acid ester of a cellulose ether is prepared from a hydroxypropoxyl-containing cellulose ether having a lower viscosity than 5 centipoise and used as a material of enteric coating of a solid medicament form, the coating film may be poor in respect of the properties as a polymeric material with increased fragility so that cracks are frequently formed in the coating film during or after the coating procedure or the coating film is partly dissolved in the fluid so that the coated solid medicament form is swollen or disintegrated in the first fluid of the pharmacopoeia. A coated solid medicament form which cannot fully withstand the attack of the first fluid, i.e. simulated gastric juice, may be disintegrated in the stomach when the medicament form is orally administrated so that such a cellulose ether product cannot provide an enteric coating on solid medicament forms.

The esterifying reagent to react with the above described hydroxypropoxyl-containing cellulose ether is an anhydride of a dicarboxylic acid or a mixture thereof with an anhydride of an aliphatic monocarboxylic acid. The dicarboxylic acid anhydride suitable for the purpose is exemplified by phthalic anhydride and succinic anhydride respectively giving an acidic phthalate and acidic succinate of the cellulose ether. When the esterifying reagent in the inventive method is a mixture of the above mentioned dicarboxylic acid anhydride and an anhydride of an aliphatic monocarboxylic acid, the resultant esterified cellulose ether is a mixed ester having two types of ester groups including the acidic dicarboxylate groups and the aliphatic acyl groups. The anhydride of aliphatic monocarboxylic acid to be combined with the dicarboxylic acid anhydride is exemplified by the anhydrides of acetic, propionic, butyric, valeric and lauric acids. Such a combined use of an aliphatic monocarboxylic acid anhydride gives an advantage that the resultant cellulose ether product may have further improved film-formability as well as the increased possibility of controlling the pH value in a wider range at which the cellulose ether product is soluble.

The esterification reaction of the above described cellulose ether as the base material with the esterifying reagent is performed by bringing them into contact in the presence of an alkali metal acetate and acetic acid in combination. The weight proportion of the reactants, i.e. the starting cellulose ether and the esterifying reagent, should adequately be selected depending on the properties of the desired product or, in particular, on the degree of esterification, i.e. the amount of the ester groups. Usually, the esterifying reagent is used in an amount in the range from 50 to 200 parts by weight per 100 parts by weight of the starting cellulose ether. When the esterifying reagent is a combination of a dicarbozylic acid anhydride and an aliphatic monocarboxylic acid anhydride, the esterifying reagent should be composed of at least 15 % by weight of the former anhydride and up to 85 % by weight of the later anhydride. The esterification reaction is performed by heating the mixture at a temperature in the range from 60 to 90 °C and the reaction is complete usually within 2 to 8 hours depending on the reaction temperature.

The alkali metal acetate used in the esterification reaction of the cellulose ether serves as a catalyst of the reaction. Suitable alkali metal acetate is exemplified by sodium and potassium acetates. The amount of the alkali metal acetate in the reaction mixture should preferably be in the range from 50 to 150 parts by weight per 100 parts by weight of the hydroxypropoxyl-containing cellulose ether as the starting material. The reaction can smoothly proceed in acetic acid as the solvent or reaction medium and the amount of acetic acid in the reaction mixture should preferably be in the range from 200 to 700 parts by weight per 100 parts by weight of the starting cellulose ether.

The reaction mixture after completion of the esterification reaction in the above described manner is a viscous liquid. When the viscous reaction mixture after the reaction is added to a large volume of water, the desired acidic dicarboxylic acid ester of cellulose ether is precipitated in the mixture and can be obtained in a powdery form by collecting the precipitates by filtration followed by thorough washing with water to remove impurities and drying.

The product obtained in the above described manner is useful as an enteric coating material on solid medicament forms. The procedure of coating with the esterified cellulose ether may be conventional. For example, a coating liquid is prepared by dissolving the cellulose ether in a solvent such as a mixture of methylene chloride and ethyl alcohol, acetone, aqueous ethyl alcohol and the like or dispersing or suspending the cellulose ether in a finely divided powdery form in water. The coating amount on solid medicament forms should preferably be in the range from 5 to 15 parts by weight per 100 parts by weight of tablets or from 20 to 30 parts by weight per 100 parts by weight of granules. The coating film formed on the solid medicament forms is very stable against the attack of the first liquid of the pharmacopoeia used in the disintegration test.

In the following, the method of the present invention is described in more detail by way of examples.

Example 1.

A reaction mixture was prepared in a reaction vessel equipped with a stirrer by introducing 100 parts by weight of a hydroxypropyl methyl cellulose containing 10 % by weight of hydroxypropoxyl groups and 29 % by weight of methoxyl groups, 120 parts by weight of phthalic anhydride, 80 parts by weight of sodium acetate and 300 parts by weight of acetic acid and the mixture was heated at 85 °C for 3.5 hours under agitation. Thereafter, 1200 parts by weight of water were added to the reaction mixture and the precipitates formed in the mixture were collected by filtration and repeatedly washed with water until the washings were no longer acidic followed by drying at 60 °C for 5 hours. Table 1 below shows the viscosity of the starting cellulose ethers, i.e. viscosity of a 2 % aqueous solution at 20 °C, and the content of carboxybenzoyl groups in the esterified products of hydroxypropyl methyl cellulose phthalate.

The esterified product was shaped into a film of 0.1 mm thickness by dissolving in a 8:2 by volume mixture of acetone and ethyl alcohol and casting the solution. The film was cut by punching into test pieces for the tensile test and the tensile strength and ultimate elongation thereof were determined on an automatic tensile tester (Autograph Model DSS-10T-S, manufactured by Shimadzu Seisakusho Co.). The results are shown in Table 1.

The esterified product was subjected to test coating of tablets and the coated tablets were subjected to the disintegration test according to Japanese Pharmacopoeia. Thus, a coating liquid was prepared by dissolving the esterified cellulose ether in a 8:2 by volume mixture of acetone and ethyl alcohol. The concentration of the coating liquid was 6 % by weight or 8 % by weight as is shown in Table 1. A tablet coating machine (24-inch Accela-coater) was charged with 10 kg of simulated tablets shaped of a mixture of lactose and starch each having a diameter of 6 mm and a weight of 90 mg and the tablets were coated with the coating liquid until the increment of the weight was 7.5 mg per tablet.

The disintegration test of the thus coated tablets was performed using 60 tablets per test and the number of disintegrated tablets was counted after 2 hours in the first fluid of the pharmacopoeia. The results are shown in Table 1.

Table 1

| Viscosity of starting cellulose ether, cps | | 3 | 6 | 15 |
|---|---|---|---|---|
| Content of carboxyben-zoyl groups, % by weight | | 33 | 32 | 33 |
| Tensile test | Tensile strength, kg/mm$^2$ | *) | 6 to 7 | 5 to 6 |
| | Ultimate elongation. % | *) | 4 to 6 | 9 to 11 |
| Concentration of coating liquid, % by weight | | 8 | 8 | 6 |
| Number of disintsegrated tablets in the first fluid | | 15 | 0 | 0 |

*) Tensile test could not be completed because of the very small cracks in the test pieces fromed in punching.

Example 2.

The procedure of the esterification reaction was substantially the same as in Example 1 except that 120 parts by weight of phthalic anhydride were replaced with a combination of 25 parts by weight of succinic anhydride and 38 parts by weight of acetic anhydride. The thus obtained hydroxypropyl methyl cellulose acetate succinate was pulverized in a jet mill into a fine powder having an average particle diameter of 6 μm. Table 2 below shows the viscosity of the starting hydroxypropyl methyl cellulose and the contents of the acidic succinoyl groups and acetyl groups in the esterified product.

The finely pulverized esterified product was shaped into a film by dissolving in acetone and casting the solution and the film was subjected to the tensile test in the same manner as in Example 1 to give the results shown in Table 2.

Table 2

| Viscosity of starting cellulose, ether, cps | | 3 | 6 |
|---|---|---|---|
| Content of acidic succinoyl groups, % by weight | | 9 | 10 |
| Content of acetyl groups, % by weight | | 10 | 10 |
| Tensile test | Tensile strength, kg/mm2 | 5 to 6 | 5 to 6 |
| | Ultimate elongation. % | 2 to 3 | 7 to 10 |
| Number of disintsegrated tablets in the first fluid | | 12 | 0 |

CLAIMS

1.    A method for the preparation of an enteric-soluble acidic dicarboxylic acid ester of a cellulose ether which comprises reacting (a) a cellulose ether having hydroxypropoxyl groups as the ether-forming groups, of which a 2 % by weight aqueous solution has a viscosity of at least 5 centipoise at 20 °C, with (b) a dicarboxylic acid anhydride or a mixture thereof with an anhydride of an aliphatic monocarboxylic acid in the presence of (c) a combination of an alkali metal acetate and acetic acid.

2.    The method as claimed in claim 1 wherein the cellulose ether having hydroxypropoxyl groups as the ether-forming groups is a hydroxypropyl cellulose or a hydroxypropyl methyl cellulose.

3.    The method as claimed in claim 1 wherein the dicarboxylic acid anhydride is phthalic anhydride or succinic anhydride.

4.    The method as claimed in claim 1 wherein the anhydride of an aliphatic monocarboxylic acid is acetic anhydride.

5.    The method as claimed in claim 1 wherein the alkali metal acetate is sodium acetate or potassium acetate.

6.    The method as claimed in claim 1 wherein the reaction is performed at a temperature in the range from 60 to 90 °C.

7. The method as claimed in claim 1 wherein the amount of the alkali metal acetate is in the range from 50 to 150 parts by weight per 100 parts by weight of the cellulose ether.

8. The method as claimed in claim 1 wherein the amount of acetic acid is in the range from 200 to 700 parts by weight per 100 parts by weight of the cellulose ether.

9. The method as claimed in claim 1 wherein the cellulose ether contains at least 4 % by weight of hydroxypropoxyl groups.